# EUROPÄISCHE PATENTSCHRIFT

(11) **EP 2 263 740 B1**
(45) Veröffentlichungstag und Bekanntmachung des Hinweises auf die Patenterteilung: **09.05.2012**
(21) Anmeldenummer: 10008956.4
(22) Anmeldetag: 30.04.2001
(51) Int. Cl.: A61N 1/05

(54) **Mikrokontaktstruktur zur Implantation bei einem Säugetier, insbesondere bei einem Menschen**
Microcontact structure for implantation in a mammal, especially a human being
Structure de microcontacts à implanter chez un mammifère, notamment chez l'homme

(30) Priorität: 28.04.2001 DE 10120908
(43) Veröffentlichungstag der Anmeldung: 22.12.2010
(62) Teilanmeldung aus: 06012895.6
(73) Patentinhaber: IMI Intelligent Medical Implants AG, 6304 Zug (CH)
(72) Erfinder: Eckmiller, Rolf, 41460 Neuss (DE); Suchert, Steffen, 90402 Nürnberg (DE)
(74) Vertreter: Graf von Stosch, Andreas

(56) Entgegenhaltungen:
- EP-A- 0 460 320
- WO-A-96/39221
- DE-A- 19 705 987
- US-A- 5 109 844
- US-A- 5 873 901

## Beschreibung

Die vorliegende Erfindung betrifft eine zur Implantation bei einem Säugetier, insbesondere beim Menschen, vorgesehene Mikrokontaktstruktur, die zur Kontaktierung von Nervengewebe im visuellen System vorgesehen ist. Genauer gesagt soll die Mikrokontaktstruktur zur Kontaktierung der Ganglienzellen in der Netzhaut um die Fovea herum einerseits oder zur Kontaktierung des visuellen Kortex (Area V1) eingerichtet werden.

Durch einen genetischen Defekt ausgelöste oder erworbene Erblindungen können unter anderem zwei Ursachen haben. Die erste Ursache ist die Zerstörung der Photorezeptorschicht in der Retina, wonach auftreffende Photonen nicht in entsprechende Stimulation der Ganglienzellen umgesetzt wird. Bei diesem Krankheitsbild sind die Ganglienzellen nur teilweise betroffen, so daß eine externe Stimulation der noch vorhandenen Ganglienzellen eine visuelle Wahrnehmung erzeugen kann. Auf dieser Basis werden seit einiger Zeit Entwicklungen vorgenommen, die die Implantation einer Mikrokontaktstruktur zur Kontaktierung der Ganglienzellen beinhalten.

Die zweite im vorliegenden Zusammenhang wesentliche Ursache der Erblindung kann in einer Unterbrechung der Signalübermittlung zwischen den Ganglienzellen und dem für die visuelle Wahrnehmung zuständigen Bereich im Gehirn oder einer Fehlfunktion der Ganglienzellen selbst liegen. Für dieses Krankheitsbild werden ebenfalls Implantate entwickelt, bei denen eine Mikrokontaktstruktur unmittelbar mit dem visuellen Kortex, genauer gesagt der Area V1 des visuellen Kortex, in Kontakt stehen und dort durch elektrische Stimulation eine visuelle Wahrnehmung erzeugen.

Die bislang bekannten Mikrokontaktstrukturen bestehen im wesentlichen aus einem Trägermaterial, das einseitig elektrisch leitende, stift- oder nadelförmig ausgebildete Kontaktelemente trägt, die sich über die Ebene der Trägerfolie erheben. Solche Mikrokontaktstrukturen sind beispielsweise aus der US 5,109,844, der US 5,159,927, der US 5,411,540, der DE 19525570 A1 oder der EP 0460320 B1 bekannt. Bei all diesen Mikrokontaktstrukturen sind die einzelnen Kontakte gleichmäßig, d.h. mit einer konstanten Flächendichte auf der Oberfläche des Implantats verteilt. US-A-5 109 844 offenbart jedoch auch das zufällige Weglassen einzelner Mikrokontakte bei matrixförmig angeordneten Kontakten. Die Flächendichte liegt dabei etwa im Bereich bis 20 Kontakten pro mm². Dabei ist man bislang davon ausgegangen, daß zur Steigerung der visuellen Auflösung eine möglichst hohe Flächendichte der Mikrokontakte erstrebenswert ist.

In der Praxis zeigt sich, daß dieses Konzept ebenso wie die bisher bekannten Mikrokontaktstrukturen problematisch ist. Ein Problem besteht darin, daß mit der zunehmenden Vergrößerung der Zahl der Mikrokontakte eine entsprechende Vergrößerung der diese Mikrokontakte speisenden externen Anordnung bedingt. Jeder einzelne Mikrokontakt wird nach den derzeitigen Konzepten von einem separaten Kanal eines Encoders gespeist. Die Randbedingungen dieser externen Vorrichtung sind die Abmessungen, der Energieverbrauch und letztlich auch die Kosten. Wenn mit einer gegebenen externen Versorgungseinheit eine bestimmte Anzahl von Mikrokontakten gespeist werden kann, so soll mit dieser Anzahl von Mikrokontakten eine möglichst optimale visuelle Wahrnehmung erzeugt werden. Hierfür ist die gegenwärtige Verteilung der Mikrokontakte mit konstanter Flächendichte nachteilig, denn sie berücksichtigt nicht, daß ein Bereich des zentralen Gesichtsfeldes für die Erkennung bestimmter Gegenstände höher zu bewerten ist als der umgebende Randbereich.

Weiter ist das mit den Mikrokontakten zu kontaktierende Gewebe nicht so aufgebaut, daß ein bestimmter Abstand zweier Punkte voneinander auf dem Gewebe in jedem Bereich des Gesichtsfeldes dem gleichen Winkelabstand bezogen auf die Blickachse entspricht. Es ist vielmehr so, daß im Falle der Ganglienzellen der Netzhaut um den Bereich des schärfsten Sehens, der Fovea genannt wird, zunächst gar keine Ganglienzellen vorhanden sind, dann in einem geringen radialen Abstand vom Mittelpunkt der Fovea eine große Dichte an Ganglienzellen vorliegt, die zu einer kraterartigen Aufwölbung am Rand der Fovea führt. Anschließend an diesen Bereich wird die Schicht der Ganglienzellen dünner.

Wird dieser Bereich mit einer Mikrokontaktstruktur belegt, die eine konstante Flächendichte der Mikrokontakte aufweist, so erreichen die Mikrokontakte im Zentrum der Fovea keine Ganglienzellen. Die mit diesen Mikrokontakten verbundenen Kanäle der externen Versorgungseinrichtung bleiben ohne physiologische Funktion. Im Bereich des "Kraterrandes" der Fovea sind pro Flächeneinheit sehr viele Ganglienzellen vorhanden, so daß für eine gegebene Anzahl von Ganglienzellen relativ wenige Mikrokontakte zur Verfügung stehen. Im Außenbereich nimmt dann die Flächendichte der Ganglienzellen ab, während die Flächendichte der Mikrokontakte gleich bleibt. Die Zahl der Mikrokontakte, die für eine bestimmte Anzahl von Ganglienzellen verfügbar sind, steigt somit an.

Diese physiologischen Gegebenheiten führen dazu, daß ausgerechnet in dem Bereich, der für das zentrale Gesichtsfeld und den Bereich des schärfsten Sehens zuständig ist, nämlich dem "Kraterrand" der Fovea, relativ wenige Mikrokontakte zur Verfügung stehen, während in dem umgebenden Bereich, der für die visuelle Wahrnehmung eine eher untergeordnete Rolle spielt, proportional viele Mikrokontakte zur Verfügung stehen. Das mit einer solchen Mikrokontaktstruktur mit konstanter Flächendichte erreichte Ergebnis ist deshalb nicht optimal, sowohl hinsichtlich des erzielten visuellen Eindrucks, der den wichtigsten Teil des Gesichtsfeldes unterrepräsentiert, als auch hinsichtlich der möglichst effektiven Nutzung der externen Versorgungseinrichtung. Entsprechendes gilt für die Kontaktierung des visuellen Kortex. Auch dort ist die Zuordnung zwischen der Oberfläche von korrespondierenden Gesichtsfeldorten auf der Hirnoberfläche und dem abgebildeten Gesichtsfeld nicht linear. Eine Mikrokontaktstruktur mit konstanter Flächendichte der Mikrokontakte würde auch hier bezogen auf die physiologische Bewertung des Gesichtsfeldes zu einer Unterrepräsentierung des zentralen Bereichs des Gesichtsfeldes führen.

Es ist deshalb Aufgabe der vorliegenden Erfindung, eine Mikrokontaktstruktur zu schaffen, die eine Kontaktierung des Nervengewebes unter Berücksichtigung der nicht linearen Zuordnung zwischen Gewebeoberfläche und dem Gesichtsfeld erlaubt und die es weiter ermöglicht, bei einer beschränkten Anzahl zur Verfügung stehender Kanäle diese so mit dem Gewebe verbindet, daß der physiologisch höher bewertete Teil des Gesichtsfeldes mit einer größeren Anzahl von Mikrokontakten abgebildet wird als der niedriger bewertete Teil des Gesichtsfeldes.

Diese Aufgabe wird für die Implantation im Auge von einer Mikrokontaktstruktur mit den Merkmalen des Anspruchs 1 und für die Implantation am visuellen Kortex von einer Mikrokontaktstruktur mit den Merkmalen des Anspruchs 7 gelöst.

Weil bei der Mikrokontaktstruktur zur Implantation im Auge über die Fläche der Mikrokontaktstruktur die Flächendichte der Mikrokontakte nicht konstant ist, kann die Verteilung der Mikrokontakte so gewählt werden, daß eine möglichst effiziente Zuordnung der verfügbaren Kanäle, die durch die externen Ressourcen begrenzt sind, zu dem abzubildenden Gesichtsfeld erfolgt. Dabei erhalten Bereiche des Gesichtsfeldes mit höherer physiologischer Bewertung eine höhere Mikrokontaktdichte als Bereiche mit geringerer Relevanz. Zusätzlich kann die Flächendichte der Mikrokontakte an die Dichte der zu kontaktierenden Neuronen in dem jeweils kontaktierten Gewebe angepaßt werden.

Vorteilhaft ist es dabei, wenn bei der Mikrokontaktstruktur zur epiretinalen Kontaktierung eine runde oder ovale rotationssymmetrische Konfiguration mit einem Mittelpunkt gewählt wird, wobei der Mittelpunkt im implantierten Zustand im Bereich der Fovea des Auges angeordnet ist und ein Bereich mit einem Radius von etwa 175 bis 200 µm um diesen Mittelpunkt frei von Mikrokontakten, zumindest von aktiv genutzten Mikrokontakten ist.

Die Mikrokontakte werden vorzugsweise mit ihrer größten verfügbaren Flächendichte in einem radial von dem Mittelpunkt um etwa 200 µm bis 1200 µm beabstandeten Bereich angeordnet. In einem radial vom Mittelpunkt um mehr als etwa 1200 µm beabstandeten Bereich, der physiologisch geringer bewertet wird, weisen die Mikrokontakte vorzugsweise eine geringere Flächendichte als im ersten Bereich auf, so daß für diesen Bereich weniger Ressourcen der externen Versorgungseinrichtung erforderlich sind.

Vorzugsweise sind die Mikrokontakte im wesentlichen rotationssymmetrisch um den Mittelpunkt der Mikrokontaktstruktur angeordnet.

Die Verteilung der Mikrokontakte in dem ersten Bereich und in dem zweiten Bereich wird vorteilhaft kontinuierlich variiert, wobei in dem ersten Bereich ein Flächendichtemaximum liegt und von da aus die Flächendichte radial nach außen kontinuierlich abfällt. Angesichts der begrenzten Zahl der Mikrokontakte (wenige 100 pro Implantat) kann auch von einer quasikontinuierlichen Verteilung gesprochen werden.

Eine Optimierung zwischen dem physiologischen Nutzen der durch die Mikrokontaktstruktur erzeugten visuellen Wahrnehmung und der Anzahl der verfügbaren Mikrokontakte bzw. der mit den Mikrokontakten verbundenen Ressourcen wird erfindungsgemäß erreicht, wenn die Flächendichte der Mikrokontakte in der Ebene, die im natürlichen Gesichtsfeld die Horizontale darstellt, größer ist als in den Bereichen, die im natürlichen Gesichtsfeld oben und unten liegen.

Entsprechendes gilt für die Mikrokontalktstruktur zur Implantation am visuellen Kortex. Dort werden die Mikrokontakte entsprechend der retinotopen Projektion des Gesichtsfeldes auf den visuellen Kortex, genauer gesagt auf die Area V1 je Gesichtsfeldhälfte, die Mikrokontakte vorzugsweise in einem etwa parabelförmigen Bereich angeordnet, wobei die Flächendichte der Mikrokontakte im Bereich des Scheitelpunktes der Parabel ein Maximum aufweist. Dabei entspricht der parabelförmige Bereich vorzugsweise der Form des visuellen Kortex, wobei die größte Dichte der Mikrokontakte in einem Bereich liegt, der der Fovea entspricht. Bevorzugt werden etwa 90% der Mikrokontakte in dem Bereich angeordnet, der einem etwa konzentrischen Gesichtsfeld mit einem Öffnungswinkel von 5° von der Blickachse entspricht.

Die Flächendichte der Mikrokontakte nimmt ausgehend von dem der Fovea zugeordneten Bereich vorzugsweise in jede Richtung kontinuierlich bzw. quasikontinuierlich ab. Dabei wird auch hier eine besonders gute Ausnutzung der Ressourcen erreicht, wenn die Flächendichte in der die Horizontalebene des natürlichen Gesichtsfeldes abbildenden Richtung höher ist als in den Bereichen, die im natürlichen Gesichtsfeld oben und unten darstellen. Bezüglich der retinotopen Projektion bedeutet das, daß entlang einer Linie von dem der Fovea entsprechenden Punkt entlang der Symmetrieachse der Parabel in temporaler und weiter exzentrischer Richtung die Flächendichte der Mikrokontakte abnimmt.

Im folgenden werden zwei Ausführungsbeispiele der vorliegenden Erfindung anhand der Zeichnung erläutert. Es zeigen:
- Fig. 1:: eine Mikrokontaktstruktur mit etwa rotationssymmetrischer Anordnung für die epiretinale Implantation in einer Draufsicht;
- Fig. 2:: eine grafische Darstellung des Verlaufs der Flächendichte der Mikrokontakte in radialer Richtung;
- Fig. 3:: eine Mikrokontaktstruktur mit etwa parabelförmiger Anordnung für die kortikale Implantation, ebenfalls in einer Draufsicht; sowie
- Fig. 4:: eine grafische Darstellung des Verlaufs der Flächendichte der Mikrokontakte in Richtung der Symmetrieachse der parabelförmigen Vorrichtung gemäß Fig. 3.

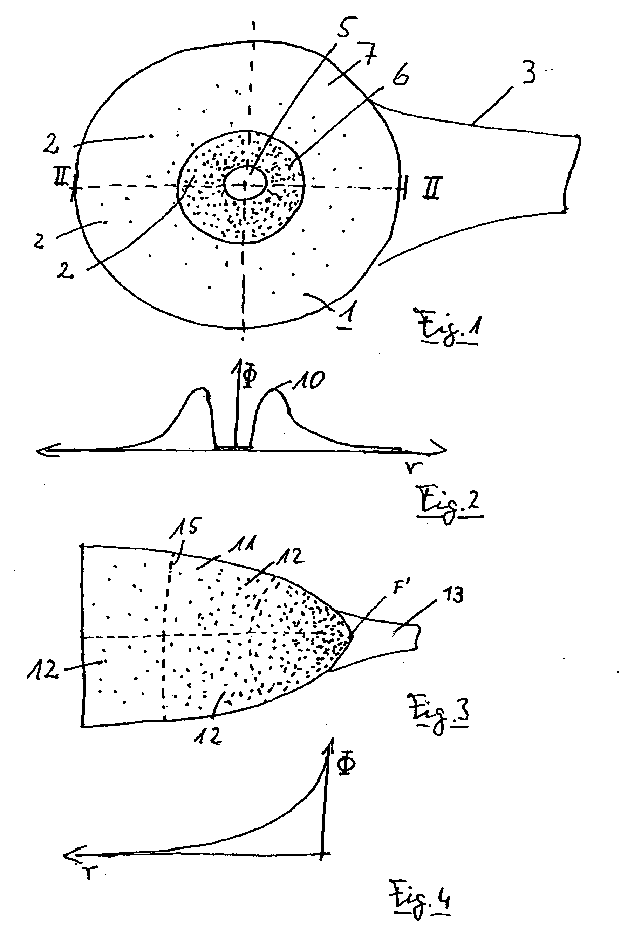

Die Figur 1 zeigt eine Mikrokontaktstruktur mit etwa rotationssymmetrischer Anordnung für die epiretinale Implantation in einem Auge in einer Draufsicht.

Die Mikrokontaktstruktur weist eine etwa kreisscheibenförmige Trägerfolie 1 auf, die beispielsweise in mehrere Sektoren gegliedert und aus Polyimid gefertigt ist. Auf dieser Trägerfolie ist eine Vielzahl (in der Fig. 1 etwa 200) von Mikrokontakten 2 ausgebildet, die in an sich bekannter Weise beispielsweise aus kleinen vorgewölbten Platinstrukturen bestehen, die etwa senkrecht auf der Trägerfolie stehen. Diese Mikrokontakte 2 sind wiederum über Leiterbahnen mit einer nach außen führenden Datenleitung 3 verbunden, die unidirektional oder bidirektional Signale an die Mikrokontakte 2 leitet bzw. von diesen empfängt. Statt einer Datenleitung kann auch eine drahtlose Kommunikation vorgesehen sein.

Die Trägerfolie 1 weist insgesamt drei konzentrische Bereiche auf, einen zentralen Bereich 5, der im implantierten Zustand die Fovea überdeckt, einen ersten Bereich 6, der im implantierten Zustand den kraterförmigen Rand der Fovea bedeckt, und einen äußeren Bereich 7, der die radial weiter außen liegenden Bereiche der Retina bedeckt.

Der Bereich 5 kann in einem konkreten Ausführungsbeispiel auch frei von Material sein, also ein mittiges Loch in dem Implantat bilden. Dieser Bereich 5 ist für das Implantat im vorliegenden Zusammenhang ohne Funktion.

Der erste Bereich 6 kontaktiert den "Kraterrand", also den die Fovea unmittelbar umgebenden Bereich, in dem die Dichte der Ganglienzellen am größten ist. Hier ist auch derjenige Bereich der Ganglienzellen angeordnet, der dem zentralen fovealen und parafovealen Gesichtsfeld entspricht. Deshalb sind in diesem Bereich 6 die Mikrokontakte 2 mit der größten Flächendichte angeordnet. Von den rund 200 Mikrokontakten in der Fig. 1 enthält der Bereich 6 etwa 180. Er repräsentiert ein Gesichtsfeld bis zu einer Exzentrizität von etwa 8° (kegelförmig mit einem Öffnungswinkel von 16°).

Der Bereich 6 wird in einer konkreten Ausführungsform nicht plan sein, sondern der Außenkontur des fovealen Bereichs der Retina angepasst und gegebenenfalls in Sektoren unterteilt sein.

Die Fig. 2 zeigt den idealisierten funktionalen Zusammenhang zwischen dem radialen Abstand eines Punktes auf der Mikrokontaktstruktur von ihrem Mittelpunkt und der Flächendichte Φ der Mikrokontakte. Es ist erkennbar, dass der zentrale Bereich 5 frei von Mikrokontakten ist, folglich die Flächendichte 0 aufweist. Der erste Bereich 6 hat bei 10 ein Maximum, was möglichst genau mit dem Bereich der höchsten Ganglienzelldichte der Retina übereinstimmt. Der zweite äußere Bereich 7 schließlich weist eine abfallende Flächendichte auf, die auch die physiologisch geringere Signifikanz des zugeordneten äußeren Gesichtsfeldes widerspiegelt. Im Betrieb soll der erste Bereich 6 mit seiner höheren Auflösung dem Implantatträger ein Erkennen eines Gegenstandes ermöglichen, während der zweite Bereich mit seiner geringeren Auflösung insbesondere eine Bewegungswahrnehmung ermöglichen soll.

Die Fig. 3 zeigt eine Draufsicht auf ein kortikales Implantat 11 mit Mikrokontakten 12, die auf der Oberfläche wie in dem Ausführungsbeispiel nach Fig. 1 senkrecht zu der Ebene der Trägerfolie angeordnet sind. Eine uni- oder bidirektionale Datenleitung 13 stellt wie in Fig. 1 die Verbindung mit einer externen Ressource her.

Die Form des Implantats entspricht der Form des rechten visuellen Kortex im Gehirn eines Rhesusaffen, in dem das Gesichtsfeld auf einen parabelförmigen Bereich abgebildet wird. Diese retinotope Abbildung des Gesichtsfeldes auf die Area V1 des visuellen Kortex ist aus der Literatur bekannt und experimentell nachgewiesen, siehe Tootell et al., J. Neur. Sci. 1988 Bd. 8 S. 1531 - 1568. Analog wird das Gesichtsfeld anderer Säugetiere ebenfalls auf den jeweiligen visuellen Kortex abgebildet, dessen Form unterschiedlich sein kann.

Zu dem dargestellten Implantat, das den rechten visuellen Kortex kontaktiert, ist ein zweites spiegelbildliches Implantat vorgesehen, das den gegenüber liegenden linken visuellen Kortex kontaktiert. Hiermit sind dann beide Gesichtsfeldhälften zugänglich.

Die Flächendichte der Mikrokontakte ist in der Figur 4 in einer Darstellung entsprechend Fig. 2 veranschaulicht. Ausgehend von dem Scheitelpunkt der Parabel, der mit F' bezeichnet ist und der der Fovea der Retina entspricht, nimmt die Flächendichte kontinuierlich ab, wobei ein sich von dem Punkt F' bis etwa zu der Linie 15 erstreckender Bereich ein Gesichtsfeld bis zu einer Exzentrizität von 5° für die zugehörige linke Gesichtfeldhälfte repräsentiert. Dieser Bereich enthält rund 80% der verfügbaren Mikrokontakte 12.

Für den Implantatträger bedeutet diese Konfiguration, dass wie oben beschrieben, der zentrale Bereich bis zu einer Exzentrizität von beispielsweise 5° das Erkennen von Gegenständen erlaubt, während der außerhalb dieses Kegels liegende Bereich größerer Exzentrizität nur eine Wahrnehmung erlaubt, die den Implantatträger möglicherweise veranlasst, sich dem peripher wahrgenommenen Objekt zuzuwenden, um es erkennen zu können.

Bei einer beschränkten Anzahl von verfügbaren stimulierenden Kanälen in der externen Ressource, von denen man in der Realität ausgehen muß, wird mit einer nicht gleichförmigen Verteilung der Mikrokontakte über die Fläche der Mikrokontaktstruktur so ein besseres Ergebnis hinsichtlich Wahrnehmung und Erkennung von Objekten und Personen erzielt als es mit den bisher bekannten Mikrokontaktstrukturen mit gleichförmiger Flächendichte der Mikrokontakte möglich ist.

Es sind neben den beschriebenen Verteilungen auch andere Konfigurationen möglich" beispielsweise mit einer noch größeren Konzentration im Bereich der Fovea, die eventuell das Lesen von Texten ermöglicht, aber bei gegebener Anzahl von extern versorgten Kanälen nur eine geringere periphere Wahrnehmung erlaubt. Im Straßenverkehr kann eine Verteilung mit anderen Schwerpunkten vorteilhaft sein, die eine für Fußgänger wichtige periphere Wahrnehmung betont, zu Lasten einer hohen Auflösung im zentralen Gesichtsfeld.

Die Mikrokontakte selbst können in Bereichen mit großer Flächendichte spitz oder mit besonders kleinem Durchmesser ausgebildet sein, während in den Außenbereichen mit geringer Flächendichte an Mikrokontakten diese eine stumpfere Ausbildung haben können, beispielsweise halbkugelförmig. So werden in den ersten Bereichen die zugeordneten rezeptiven Felder sehr selektiv stimuliert, während im Außenbereich größere rezeptive Felder mit wenigen Mikrokontakten erreicht werden.

Während in den Ausführungsbeispielen beschrieben wurde, dass die tatsächlich vorhandene Anzahl der Mikrokontakte pro Flächeneinheit variiert, können bei anderen Ausführungsformen auch Mikrokontakte mit gleichmäßiger Flächendichte vorhanden sein, wenn die Zahl der aktiv mit der externen Ressource verbundenen Mikrokontakte variiert. Im peripheren Bereich wird dann eine größere Zahl von Mikrokontakten zwar physisch vorhanden sein, aber nicht kontaktiert sein, während im Bereich des zentralen Gesichtsfeldes jeder oder nahezu jeder Mikrokontakt aktiv ist.

## Patentansprüche

1. Mikrokontaktstruktur zur epiretinalen Kontaktierung von Nervengewebe im Auge für eine Sehprothese bei Säugetieren oder Menschen, wobei über die Fläche der Mikrokontaktstruktur die Flächendichte der Mikrokontakte (2) nicht konstant ist, **dadurch gekennzeichnet, dass** die Flächendichte der Mikrokontakte (2) in der Ebene die im natürlichen Gesichtsfeld der Horizontale darstellt, grösser ist als in den Bereichen, die im natürlichen Gesichtsfeld oben und unten liegen.

2. Mikrokontaktstruktur nach Anspruch 1, **dadurch gekennzeichnet, dass** ein Mittelpunkt vorgesehen ist, der im implantierten Zustand im Bereich der Fovea des Auges angeordnet ist, und dass ein Bereich (5) mit einem Radius von etwa 175 µm bis 200 µm um dieser Mittelpunkt frei von Mikrokontakten ist.

3. Mikrokontaktstruktur nach einem der vorhergehenden Ansprüche, **dadurch gekennzeichnet, dass** die Mikrokontakte (2) mit einer größten Flächendichte in einem radial von dem Mittelpunkt um etwa 200 µm bis 1200 µm beabstandeten Bereich (6) angeordnet sind.

4. Mikrokontaktstruktur nach Anspruch 3, **dadurch gekennzeichnet, dass** die Mikrokontakte (2) in einem radial von dem Mittelpunkt um mehr als etwa 1200 µm beabstandeten zweiten Bereich (7) eine geringere Flächendichte als in dem ersten Bereich (6) aufweisen.

5. Mikrokontaktstruktur nach einem der vorhergehenden Ansprüche, **dadurch gekennzeichnet, dass** die Mikrokontakte im wesentlichen rotationssymmetrisch um den Mittelpunkt der Mikrokontaktstruktur angeordnet sind.

6. Mikrokontaktstruktur nach Anspruch 4, **dadurch gekennzeichnet, dass** die Verteilung der Mikrokontakte in dem ersten Bereich (6) und dem zweiten Bereich (7) in Radialrichtung kontinuierlich variiert, wobei in dem ersten Bereich (6) ein Flächendichtemaximum liegt und von da aus die Flächendichte radial nach außen kontinuierlich abfällt.

7. Mikrokontaktstruktur zur Kontaktierung von Nervengewebe des visuellen Kortex für eine Sehprothese bei Säugetieren oder Menschen, **dadurch gekennzeichnet, dass** über die Fläche der Mikrokontaktstruktur die Flächendichte der Mikrokontakte (12) nicht konstant ist, wobei die Flächendichte der Mikrokontakte (12) in der Ebene, die im natürlichen Gesichtsfeld die Horizontale darstellt, größer ist als in den Bereichen, die im natürlichen Gesichtsfeld oben und unten liegen.

8. Mikrokontaktstruktur nach Anspruch 7, **dadurch gekennzeichnet, dass** die Mikrokontakte (12) in einem etwa der retinotopen Projektion des zentralen Gesichtsfeldes angepassten Bereich angeordnet sind, wobei die Flächendichte der Mikrokontakte im Bereich der Projektion der Fovea ein Maximum aufweist.

9. Mikrokontaktstruktur nach einem der Ansprüche 7 oder 8, **dadurch gekennzeichnet, dass** der kontaktierte Bereich der Form des visuellen Kortex entspricht.

10. Mikrokontaktstruktur nach Anspruch 8, **dadurch gekennzeichnet, dass** die Flächendichte der Mikrokontakte 12 ausgehend von dem der Fovea zugeordneten Bereich in Richtung zunehmender Exzentrizität kontinuierlich abnimmt.

## Claims

1. Microcontact structure for epiretinal contacting of nerve tissue in the eye for a vision prosthesis in mammals or human beings, wherein the surface density of the microcontacts (2) is not constant over the surface of the microcontact structure, **characterised in that** the surface density of the microcontacts (2) in the plane representing the horizontal in the natural visual field is greater than in the regions which are located at the top and bottom in the natural visual field.

2. Microcontact structure according to claim 1, **characterised in that** a central point is provided which, in the implanted state, is arranged in the region of the fovea of the eye, and **in that** a region (5) with a radius of about 175 µm to 200 µm is free of microcontacts around this central point.

3. Microcontact structure according to any one of the preceding claims, **characterised in that** the microcontacts (2) with the greatest surface density are arranged in a region (6) radially spaced from the central point by about 200 µm to 1200 µm.

4. Microcontact structure according to claim 3, **characterised in that** the microcontacts (2) in a second region (7) radially spaced from the central point by more than about 1200 µm have a lower surface density than in the first region (6).

5. Microcontact structure according to any one of the preceding claims, **characterised in that** the microcontacts are substantially rotationally symmetrically arranged around the central point of the microcontact structure.

6. Microcontact structure according to claim 4, **characterised in that** the distribution of the microcontacts varies continuously in the radial direction in the first region (6) and the second region (7), a surface density maximum being located in the first region (6) and the surface density continuously decreasing radially outwardly from there.

7. Microcontact structure for contacting nerve tissue of the visual cortex for a vision prosthesis in mammals or human beings, **characterised in that** the surface density of the microcontacts (12) is not constant over the surface of the microcontact structure, wherein the surface density of the microcontacts (12) in the plane representing the horizontal in the natural visual field is greater than in the regions which are located at the top and bottom in the natural visual field.

8. Microcontact structure according to claim 7, **characterised in that** the microcontacts (12) are arranged in a region substantially adapted to the retinotopic projection of the central visual field, the surface density of the microcontacts having a maximum in the region of projection of the fovea.

9. Microcontact structure according to claim 7 or 8, **characterised in that** the contacted region corresponds to the form of the visual cortex.

10. Microcontact structure according to claim 8, **characterised in that** the surface density of the microcontacts (12) decreases continuously, starting from the region associated with the fovea, in the direction of increasing eccentricity.

## Revendications

1. Structure de microcontacts pour une mise en contact épirétinale de tissus nerveux dans l'oeil pour une prothèse visuelle chez les mammifères ou les hommes, la densité surfacique des microcontacts (2) n'étant pas constante sur la surface de la structure de microcontacts, **caractérisée en ce que** la densité surfacique des microcontacts (2) est plus grande dans le plan qui représente l'horizontale dans le champ visuel naturel que dans les zones qui se situent en haut et en bas dans le champ visuel naturel.

2. Structure de microcontacts selon la revendication 1, **caractérisée en ce qu'**il est prévu un centre qui, à l'état implanté, se situe dans la zone de la fovéa de l'oeil, et **en ce qu'**une zone (5) avec un rayon d'environ 175 µm à 200 µm autour de ce centre ne contient pas de microcontacts.

3. Structure de microcontacts selon l'une quelconque des revendications précédentes, **caractérisée en ce que** les microcontacts (2) sont agencés avec la plus grande densité surfacique dans une zone (6) écartée radialement du centre d'une distance d'environ 200 µm à 1 200 µm.

4. Structure de microcontacts selon la revendication 3, **caractérisée en ce que**, dans une deuxième zone (7) écartée radialement du centre d'une distance supérieure à environ 1 200 µm, les microcontacts (2) présentent une densité surfacique plus faible que dans la première zone (6).

5. Structure de microcontacts selon l'une quelconque des revendications précédentes, **caractérisée en ce que** les microcontacts sont agencés sensiblement par symétrie de révolution autour du centre de la structure de microcontacts.

6. Structure de microcontacts selon la revendication 4, **caractérisée en ce que** la répartition des microcontacts dans la première zone (6) et dans la deuxième zone (7) varie en continu dans le sens radial, un maximum de densité surfacique se situant dans la première zone (6) et la densité surfacique diminuant à partir de là en continu dans le sens radial vers l'extérieur.

7. Structure de microcontacts pour une mise en contact de tissus nerveux du cortex visuel pour une prothèse visuelle chez les mammifères ou les hommes, **caractérisée en ce que** la densité surfacique des microcontacts (12) n'est pas constante sur la surface de la structure de microcontacts, la densité surfacique des microcontacts (12) étant plus grande dans le plan qui représente l'horizontale dans le champ visuel naturel que dans les zones qui se situent en haut et en bas dans le champ visuel naturel.

8. Structure de microcontacts selon la revendication 7, **caractérisée en ce que** les microcontacts (12) sont agencés dans une zone adaptée à peu près à la projection rétinotopique du champ visuel central, la densité surfacique des microcontacts présentant un maximum dans la zone de projection de la fovéa.

9. Structure de microcontacts selon l'une quelconque des revendications 7 ou 8, **caractérisée en ce que** la zone mise en contact correspond à la forme du cortex visuel.

10. Structure de microcontacts selon la revendication 8, **caractérisée en ce que** la densité surfacique des microcontacts (12) diminue en continu en partant d'une zone associée à la fovéa selon une excentricité croissante.
